# EUROPEAN PATENT SPECIFICATION

(11) **EP 3 696 266 B1**
(45) Date of publication and mention of the grant of the patent: **03.04.2024**
(21) Application number: 18866494.0
(22) Date of filing: 09.10.2018
(51) Int. Cl.: A01K 67/027, C12N 5/0735, C12N 15/63, C12N 15/113

(54) **PLURIPOTENT STEM CELLS HAVING MODIFIED DIFFERENTIATION POTENTIAL**
PLURIPOTENTE STAMMZELLEN MIT MODIFIZIERTEM DIFFERENZIERUNGSPOTENTIAL
CELLULES SOUCHES PLURIPOTENTES PRÉSENTANT UN POTENTIEL DE DIFFÉRENCIATION MODIFIÉ

(30) Priority: 10.10.2017 JP 2017197034
(43) Date of publication of application: 19.08.2020
(73) Proprietor: The University of Tokyo, Bunkyo-ku, Tokyo 113-8654 (JP)
(72) Inventor: NAKAUCHI, Hiromitsu, Tokyo 113-8654 (JP); SATO, Hideyuki, Tokyo 113-8654 (JP); MASAKI, Hideki, Tokyo 113-8654 (JP); WATANABE, Motoo, Tokyo 113-8654 (JP)
(74) Representative: Hoffmann Eitle
(86) International application number: PCT/JP2018/037566
(87) International publication number: WO 2019/073960

(56) References cited:
- WO-A1-2017/175745
- POUEYMIROU WILLIAM T ET AL: "F0 generation mice fully derived from gene-targeted embryonic stem cells allowing immediate phenotypic analyses", NATURE BIOTECHNOLOGY, GALE GROUP INC, NEW YORK, vol. 25, no. 1, 2007, pages 91-99, XP002464122, ISSN: 1087-0156, DOI: 10.1038/NBT1263
- RHINN ET AL: "Sequential roles for Otx2 in visceral endoderm and neuroectoderm for forebrain and midbrain induction and specification", DEVELOPMEN, vol. 125, no. 5, 1998, pages 845-856, XP055593401,
- NAGASHIMA HIROSHI ET AL: "Growing human organs in pigs-A dream or reality?", THERIOGENOLOGY, LOS ALTOS, CA, US, vol. 86, no. 1, 21 April 2016 (2016-04-21) , pages 422-426, XP029556981, ISSN: 0093-691X, DOI: 10.1016/J.THERIOGENOLOGY.2016.04.056
- MASASHI YAMAJI ET AL: "Critical function of Prdm14 for the establishment of the germ cell lineage in mice", NATURE GENETICS, vol. 40, no. 8, August 2008 (2008-08), pages 1016-1022, XP055167610, ISSN: 1061-4036, DOI: 10.1038/ng.186
- RHINN, M. et al.: "Sequential roles for Otx2 in visceral endoderm and neuroectoderm for forebrain and midbrain induction and specification", Development, vol. 125, no. 5, 1998, pages 845-856, XP055593401,
- RASHID, T. et al.: "Revisiting the flight of Icarus: marking human organs from PSCs with large animal chimeras", Cell Stem Cell, vol. 15, no. 4, 2014, pages 406-409, XP055343901, DOI: 10.1016/j.stem.2014.09.013
- DONDORP, W. et al.: "Human-animal chimeras: circumventing rather than discussing ethical concerns comes at a price", Reprod. Biomed., vol. 35, no. 4 July 2017 (2017-07), pages 341-342, XP085206323, DOI: 10.1016/j.rbmo.2017.07.001
- YAMAJI, M. et al.: "Critical function of Prdml4 for the establishment of the germ cell lineage in mice", Nat. Genet., vol. 40, no. 8, 2008, pages 1016-1022, XP055167610, DOI: 10.1038/ng.186
- OJI, A. et al.: "CRISPR/Cas9 mediated genome editing in ES cells and its application for chimeric analysis in mice", Sci. Rep., vol. 6, no. 31666, 2016, pages 1-9, XP055695764,

## Description

### Technical Field

The present invention relates to a pluripotent stem cell having a modified differentiation potential.

### Background Art

Genetically modified animals are frequently used for analyzing *in-vivo* gene function; transgenic animals having a foreign gene introduced therein and gene-knockout animals having an endogenous gene disrupted are known.

A genetically modified animal is produced by introducing a pluripotent cell having a desired gene modification into a wild-type animal embryo or by introducing a desired gene modification directly into an animal embryo. When an animal embryo having a pluripotent cell having a desired genetic modification introduced therein is transplanted in the uterus of a false pregnant foster mother and allowed to develop, the individual develops from the animal embryo has becomes a chimeric state consisting of wild-type cells and genetically modified cells.

Non Patent Literature 1 discloses that a Prdm14 gene knockout mouse lacks a germ cell. Since the Prdm14 gene knockout mouse lacks the germ cell, it cannot be crossed.

### Citation List

### Non Patent Literature

Non Patent Literature 1: Yamaji, M. et al., Nature Genetics, 40(8): 1016-1022, 2008

### Summary of Invention

The present invention provides a pluripotent stem cell having a modified differentiation potential.

**The invention is set out in the appended set of claims.**

### Brief Description of Drawings

[Figure 1] Figure 1 shows a chimera rate of a cell introduced in a non-human embryo in a non-human somatic chimera animal, which was obtained by introducing a wild type, male or female Prdm14 gene knockout ES cell into a wild-type non-human embryo.

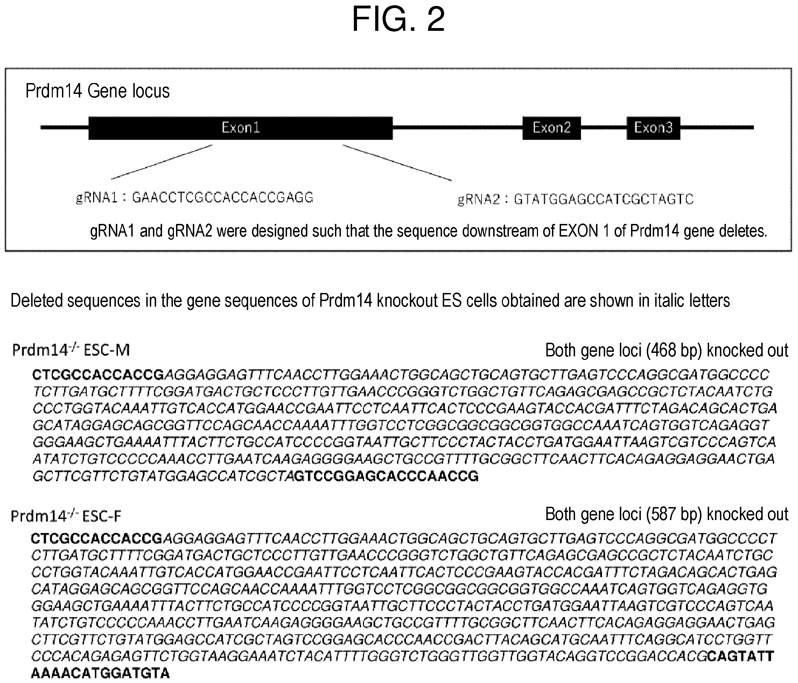

[Figure 2] Figure 2 shows a method for disrupting Prdm14 gene by CRISPR/Cas9 system, and shows Prdm14 gene disrupted.
[Figure 3A] Figure 3A shows a cell-specific cell death induction system.
[Figure 3B] Figure 3B shows a cell-specific cell death induction system.
[Figure 3C] Figure 3C shows a cell-specific cell death induction system.
[Figure 3D] Figure 3D shows a cell-specific cell death induction system.
[Figure 4] Figure 4 shows a method for preparing an Otx2 knockout ES cell.
[Figure 5] Figure 5 shows the overall distribution of a transplanted cell in a non-human individual obtained by introducing an Otx2 knockout ES cell into a wild-type non-human embryo, and shows the results of immunochemical staining of brain-tissue sections.
[Figure 6] Figure 6 shows expression of GFP in the pancreas of a non-human individual (#11), which was obtained by introducing Prdm14 knockout (Prdm14 -/-) and Otx2 knockout (Otx2 -/-) GFP expressing ES cells into a non-human Pdx1-/-embryo.
[Figure 7] Figure 7 shows that the pancreas of a non-human individual (#11) has a normal function by a glucose tolerance test; and a genotype of Pdx1 gene of a non-human fetus obtained in a crossing test of a non-human individual (#11) and a wild-type mouse.

### Description of Embodiments

As used herein, the "animal defective in own germ cell in the developmental stage" refers to a non-human animal which does not generate its own germ cell in the developmental stage. Examples of such an animal include an animal having a cell-autonomous defect, that is, an animal defective in own germ cell in the developmental stage.

As used herein, the "differentiation potential" used for a pluripotent cell refers to a potential of the pluripotent cell to differentiate into tissues present in a fetus and an adult.

As used herein, the "cell-autonomous defect" means that abnormality intrinsic to a cell substantially and quantitatively or qualitatively affects only to the cell. Preferably, the "cell autonomous defect" is not a defect affecting other cells. Since the "cell autonomous defect" is a defect having an effect only on the cell having the defect, if a non-defective cell is introduced into a host, the cell does not receive any effect from the host. Examples of the "cell autonomous defect" include modification inducing tissue-specific cell death and modification depriving a cell autonomous factor required for tissue differentiation.

As used herein, the "animal" is a non-human animal and refers to a mammal and a bird. Examples of the animal include, but are not particularly limited to, a rodent such as a mouse and a rat, a livestock animal such as a pig and a cow, a pet animal such as a dog and a cat, a bird such as a chicken, and a primate such as a monkey.

As used herein, the "intraspecies" refers to isogenic and allogenic. The "interspecies" refers to different species in the non-human animal taxonomy.

As used herein, the "somatic chimera animal" is a non-human animal and refers to an animal having a tissue or organ in which the cell of a non-human individual coexist (at the cell level) with the cell of another non-human individual (for example, isogenic cells or isogenic cells having one or more genetic modifications or allogeneic cells).

As used herein, the "host animal" is a non-human animal and refers to a non-human embryo to which pluripotent cells are to be introduced in producing a non-human somatic chimera animal.

As used herein, "the cell to be introduced into an embryo" refers to the cell to be introduced into a non-human embryo in producing a non-human somatic chimera animal.

As used herein, the "pluripotent cell" refers to a pluripotent stem cell such as an ES cell and an iPS cell, and a pluripotent cell such as inner cell mass (ICM). The pluripotent cell is known to be differentiated into any types of cells.

As used herein, "genetic engineering" refers to modification of gene expression and genetic modification.

As used herein, modification of gene expression refers to modification by which gene expression is enhanced or attenuated. Gene expression can be enhanced by connecting an endogenous gene operatively to at least one regulatory sequence to allow the endogenous gene to strongly express within the cell. Gene expression can be attenuated by a knockdown technique using an antisense nucleic acid to an endogenous gene, siRNA and a nucleic acid derivative thereof (for example, bridged nucleic acid (BNA), locked nucleic acid (LNA), a hybrid nucleic acid containing different types of nucleic acids, a peptide nucleic acid (PNA)) and shRNA and a nucleic acid derivative thereof.

As used herein, "genetic modification" means that a gene is different from a wild type gene and includes a naturally occurring modification and an artificial modification. Representative examples of the genetic modification include transgenic modification and knockout.

As used herein, "crossing" refers to fertilization between two non-human individuals in order to obtain a next generation; in other words, obtaining a non-human fetus from two non-human individuals as parents. Examples of crossing include artificial crossing by an insemination technique and pairing of a male and a female for reproduction.

The present invention provides a a pluripotent cell having a modified differentiation potential.

In an embodiment of the present invention, examples of the pluripotent cell having a modified differentiation potential include a pluripotent cell genetically engineered not to differentiate into a predetermined type of cell.

In an embodiment of the present invention, preferably, the pluripotent cell having a modified differentiation potential may have a cell autonomous defect. For example, a pluripotent cell, which has a cytotoxic gene operatively connected to an enhancer and/or a promoter of a gene that is induced when the cell differentiates into a predetermined type of cell, autonomously dies when it differentiates into the predetermined type of cell. Thus, such a pluripotent cell can be employed as the pluripotent cell having an autonomous defect and a modified differentiation potential.

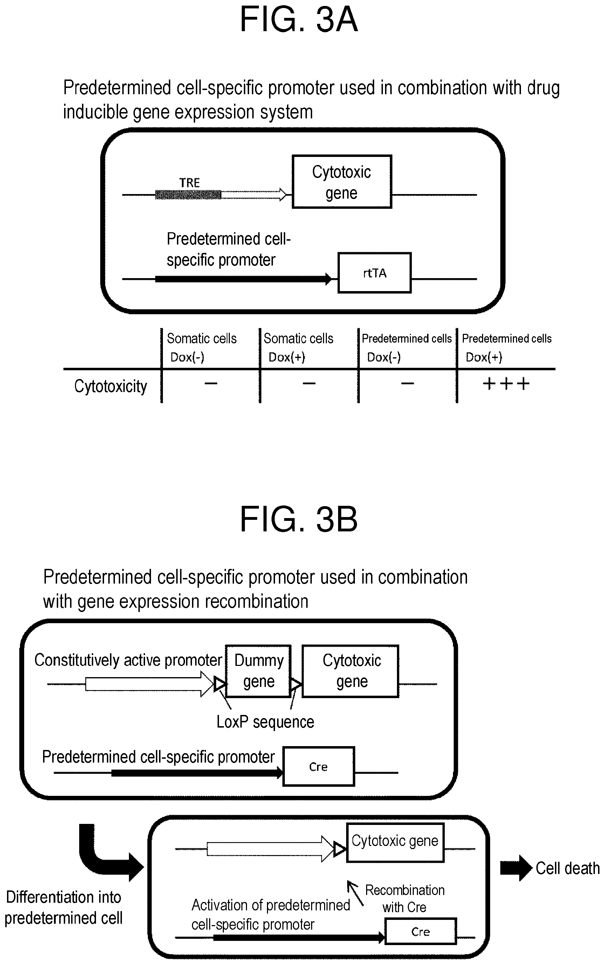

In an embodiment of the present invention, cell autonomous cell-death can be induced. Cell death can be induced by expressing a cytotoxic gene such as Caspase-8, Caspase-9, Barnase, and diphtheria toxin in a predetermined cell (such as a germ cell) depending on the concentration of a drug (for example, doxycycline) within a non-human embryo, by using, for example, a drug-inducible gene expression system (for example, tetracycline-responsive expression induction system) in combination. Now, a method for producing cell-specific cytotoxicity will be described below with reference to Figures 3A to D.

Figure 3A shows a system for inducing cell-specific and drug-concentration dependent cell-death by using a cell-specific promoter in combination with a drug-inducible gene expression system. The drug-inducible gene expression system herein is Tet-On system. In Figure 3A, a reverse tetracycline-regulated transactivator (rtTA) is operatively connected to a cellspecific promoter. A non-human fetus to which this system is integrated, expresses rtTA in a cell-specific manner. Herein, a cytotoxic gene is induced only in a cell expressing rtTA by a tetracycline compound such as doxycycline (Dox). In this way, cell-specific cell death can be induced.

Another example of the methods for attaining the same purpose is a method for expressing a cytotoxic gene in a cell-specific manner by a genetic recombination system such as Cre-LoxP. Figure 3B shows a system for inducing cell death in a cell-specific manner by using a tissue-specific gene recombination system such as Cre-LoxP. In Figure 3B, a physiological meaningless gene (dummy gene) is connected to a cytotoxic gene at the downstream of a constitutively active promoter. In this state, cell death cannot be induced in the cell. However, in this case, a LoxP sequence is each arranged at the upstream and downstream of the dummy gene. In addition, this cell has Cre operatively connected to a cell-specific promoter. In this system, when the cell is differentiated into a predetermined cell, Cre is driven by the cell-specific promoter and expressed in a cell-specific manner, and acts on the LoxP sequences to remove the dummy gene. In this manner, the cytotoxic gene is allowed to connect operatively to a constitutively activated promoter to express the cytotoxic gene in a cell-specific manner, with the result that the cell can be induced in a cell-specific manner. In this system, Cre recombinase may be replaced by CreER, which is activated by 4-tamoxifen hydroxide. CreER is known as a fusion protein of Cre recombinase and a mutant of a ligand binding region in estrogen receptor, which is activated by 4-tamoxifen hydroxide.

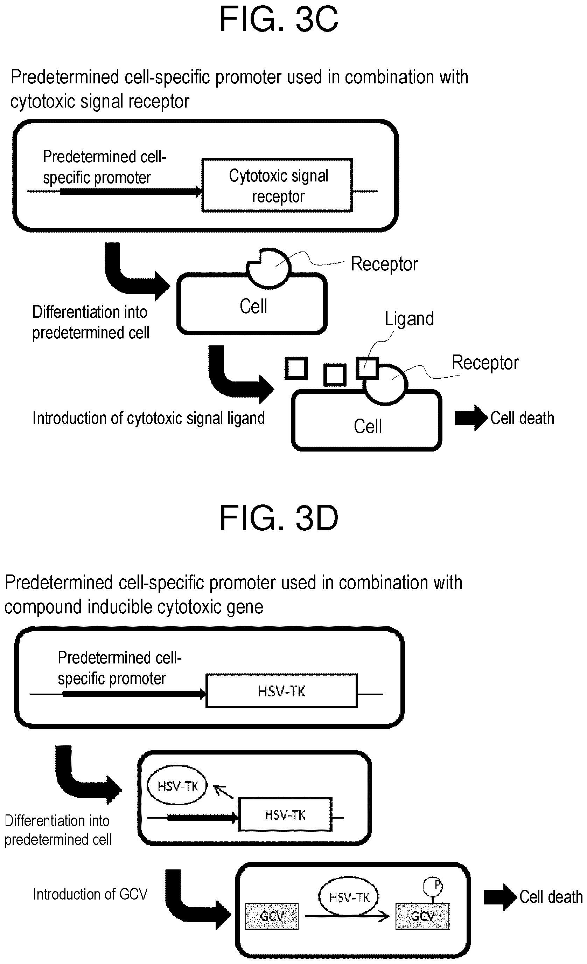

Still another example of the methods for attaining the same purpose is a method of inducing cell death in a cell-specific manner by expressing a cytotoxic-signal receptor in a cell-specific manner and allowing a ligand to act on the receptor at appropriate timing. In this case, the cell that does not express a cytotoxic signal receptor is preferably insensitive to the ligand. In Figure 3C, a system, in which a cytotoxic-signal receptor is operatively connected to a cell-specific promoter, is integrated into a cell. When the cell is differentiated into a predetermined cell, the cytotoxic signal receptor is expressed on the cell surface and cell death is induced in a ligand-dependent manner. Examples of the cytotoxic-signal receptor and a ligand thereof include a diphtheria toxin receptor and diphtheria toxin.

Yet another example of the methods for attaining the same purpose is a compound inducible cytotoxic system. Examples of other compound inducible cytotoxic system include HSV-TK/GCV system (Moolten FL et al., Hum. Gene Ther. 1990; 1: 125-134) and a system using inducible caspase-9 (Straathof KC et al., Blood 2005; 105: 4247-4254).

Figure 3D shows the HSV-TK/GCV system. To describe it more specifically, ganciclovir (GCV) not phosphorylated has a weak cytotoxicity; however, if a thymidine kinase gene (HSV-TK) that the genus herpesvirus has, is allowed to act on GCV, GCV is phosphorylated and converted into GCV triphosphate to acquire cytotoxicity. Thus, if HSV-TK is operatively connected to a cell-specific promoter and expressed in a cell-specific manner, cell death can be induced in a cell-specific manner. Inducible caspase-9 is a protein obtained by replacing CARD in caspase-9 with FKBP12, and can be dimerized and activated only in the presence of a tacrolimus derivative such as AP1903 to induce cell death. If the system, in which inducible caspase-9 is operatively connected to a cell-specific promoter, is introduced into a cell and the cell is differentiated into a predetermined cell, cell death is induced by the tacrolimus derivative in a cell-specific manner.

The aforementioned methods can be used in combination. Induction of cell-specific cell death can be attained by those skilled in the art based on the contents of the specification and the technical knowledge in the art.

For example, a cell autonomous defect can be generated in a germ cell. For example, a pluripotent cell, which has a cytotoxic gene operatively connected to an enhancer and/or a promoter of a gene expressed specifically in a germ-cell, such as Prdm14, Nanos2, Nanos3, DDX4, and Sox17, autonomously die if it is differentiated into a germ cell. Because of this, such a pluripotent cell does not contribute to a germ cell of a non-human somatic chimera animal. The gene specifically expressed in a germ cell can be, for example, a gene temporarily or long and specifically expressed in a germ cell.

For example, contribution of a pluripotent cell to the cerebral cortex can be limited. For example, a pluripotent cell, which has a cytotoxic gene operatively connected to an enhancer and/or promoter of a gene specifically expressed in a forebrain (the cerebrum in future), such as Otx1 and Otx2, and which is destined to develop into the forebrain dies. Thus the pluripotent cell does not contribute to the cerebral cortex in a non-human somatic chimera animal. A gene specifically expressed in the forebrain can be defined as a gene temporarily and long; and substantially expressed in the forebrain region. Then, disadvantage brought by contribution of the cell introduced into a non-human embryo to the brain, for example, occurrence of a damage on a higher brain function can be avoided.

In an aspect, even if Izumo gene specifically expressed in a sperm is deleted, sperms can be formed; however it is known that the sperms formed do not have a fertilization function. Deletion of such a gene may induce dysfunction of gametes.

In an aspect, a gamete may be removed by using a sperm-specific promoter/enhancer such as Izumo's one in combination with a cytotoxic gene. Similarly, using a promotor and/or enhancer of a gene such as Tpap, Acrosin and Tra98 specifically expressed in, for example, a sperm and a gene such as Gdf9, Zp1 and Zp3, specifically expressed in an egg in combination with a cytotoxic gene, a gamete can be removed in the gamete formation process.

In another aspect, formation of a germ cell can be avoided by using a promoter/enhancer of a gene such as Dazl, Stra8, Taf7l or Sycp3, involved in the meiosis that specifically occurs in a germ cell.

A cell in which a gene essential for germ cell differentiation is disrupted, can be used.

Prdm14 is a gene encoding a PR domain-containing protein 14. Prdm14 is also referred to as PR domain zinc finger protein 14. Prdm14 protein has an amino acid sequence registered, for example, under HPRD ID: 11457 and the gene thereof is present at position 8q13.3 on a chromosome. In a Prdm14 gene-knockout mouse, it is known that the germ cells in the ovary and testis are deleted (Yamaji M. et al., Nature Genetics, 40: 1016-1022, 2008). A pluripotent cell, from which Prdm14 gene is knocked out or knocked down to prevent differentiated into a germ cell, may be used as the cell. In an embodiment of the present invention, Prdm14 gene can be knocked out by, e.g., deleting a part or whole of the gene or introducing a frame shift. In an embodiment of the present invention, knockout Prdm14 gene has the sequence represented by SEQ ID NO:3 or SEQ ID NO:4.

According to the present invention, as the pluripotent cell genetically engineered not to be differentiated into a predetermined type of cell, a Prdm14 knockout and Otx2 knockout pluripotent cell (for example, ES cell and iPS cell) can be used. As described, the present invention provides an invention using a pluripotent cell genetically engineered not to differentiate into the brain and gonad as the pluripotent cell genetically engineered not to be differentiated into a predetermined type of cell.

Interestingly, as is apparent from the following Examples, in the living body, the cell introduced into a non-human embryo and a non-human host cell have high flexibility, that is, even if one of the cells cannot be differentiated into a predetermined tissue, the other cell complementarily is differentiated into the tissue. To be more specific, if the cell introduced into an non-human embryo is not differentiated into a predetermined type of cell, the non-human embryo cell of a host can be complementarily differentiated, with the result that the predetermined type of cell (differentiated from the cell introduced into a non-human embryo) forms a non-human chimera consisting of the cell introduced into a non-human embryo and the cell of a non-human host embryo. In contrast, if the cell introduced into a non-human embryo is not differentiated into a predetermined type of cell, the cell derived from a non-human host embryo consists the predetermined type of cell. A differentiation defect of the cell introduced into a non-human embryo is complemented by the non-human host embryo cell, with the result that the defect will not appear as an abnormality in the non-human somatic chimera animal.

It has been demonstrated that, in blastocyst complementation of an organ-deficient non-human animal, a defective organ is complemented by an organ consisting of the cell introduced into a non-human embryo (for example, WO2010/087459,

Accordingly, in an embodiment, as a pluripotent cell genetically engineered not to be differentiated into a predetermined type of cell, a Prdm14 knockout and Otx2 knockout pluripotent cell (for example, ES cell and iPS cell) can be used; and, as thenon-human embryo, a Pdx1-/-knockout non-human embryo or a Pdx1-Hes1 transgenic non-human embryo can be used. In an embodiment, as the pluripotent cell genetically engineered not to be differentiated into a predetermined type of cell, a Prdm14 knockout and Otx2 knockout pluripotent cell (for example, ES cells and iPS cell) can be used; and, as the non-human embryo, an Sall1 knockout non-human embryo can be used.

According to the present invention, as the pluripotent cell genetically engineered not to be differentiated into a predetermined type of cell, a pluripotent cell genetically engineered not to be differentiated into the brain and gonad can be used. The pluripotent cell genetically engineered not to be differentiated into the brain and gonad can be prepared by genetic engineering not to differentiate into the brain and genetic engineering not to differentiate into the gonad.

For example,
a pluripotent cell having both
a cytotoxic gene, which is operatively connected to an enhancer and/or promoter of a gene exhibiting germ cell specific expression, such as Prdm14, Nanos2, Nanos3, DDX4, and Sox17, and
a cytotoxic gene, which is operatively connected to an enhancer and/or promoter of a gene exhibiting forebrain (the cerebrum in future) specific expression, such as Otx1 and Otx2,
can be used as the pluripotent cell genetically engineered not to be differentiated into a predetermined type of cell.

For example,
a pluripotent cell having both
a cytotoxic gene operatively connected to an enhancer and/or promoter of a gene exhibiting gamete-specific expression, for example, a gene exhibiting a sperm-specific expression, such as Izumo, Tpap, Acrosin, or Tra98, or an egg-specifically expressing gene such as Gdf9, Zp1 or Zp3; and
a cytotoxic gene operatively connected to an enhancer and/or promoter of a gene exhibiting forebrain (the cerebrum in future) specific expression, such as Otx1 and Otx2,
can be used as the pluripotent cell genetically engineered not to be differentiated into a predetermined type of cell.

For example,
a pluripotent cell having both
a cytotoxic gene operatively connected to an enhancer and/or promoter of a gene involved in meiosis, which is a gamete-specific phenomenon, such as Dazl, Stra8, Taf7l or Sycp3; and
a cytotoxic gene operatively connected to an enhancer and/or promoter of a gene exhibiting a forebrain (the cerebrum in future) specific expression, such as Otx1 and Otx2,
can be used as the pluripotent cell genetically engineered not to be differentiated into a predetermined type of cell.

Examples of the cell are a pluripotent cell of a primate such as a human and a monkey, and, a pluripotent cell of a mammal such as pig, cow, sheep and goat. In an embodiment of the present invention, the cell is a human pluripotent cell (for example, ES cell or iPS cell).

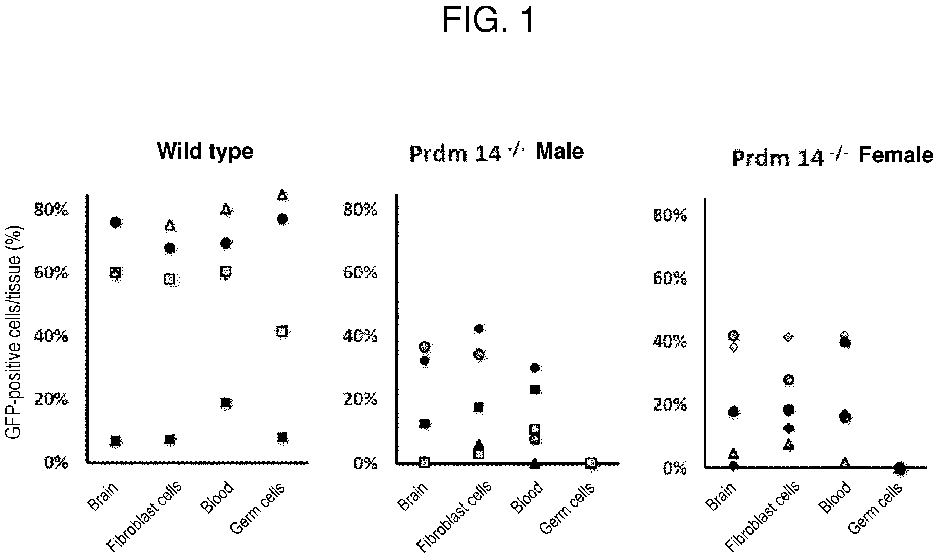

It is considered that the chimera formation ability of a pluripotent cell is different depending on type of cell (see, for example, the panel "wild type" of Figure 1). Therefore, a pluripotent cell that can provide a desired chimera except a predetermined type of cell can be selected and used. The chimera varies depending on the chimera formation ability of a pluripotent cell. If the chimera formation ability of a pluripotent cell is high, the percentage of the cells derived from the pluripotent cell is high. In contrast, if the chimera formation ability of a pluripotent cell is weak, the percentage of the cells derived from the pluripotent cell is low. The chimera formation ability can be determined by labeling the cell introduced into a non-human embryo

Accordingly, an aspect may include selecting a pluripotent cell having desired chimera formation ability and modifying the differentiation potential of the obtained pluripotent stem cell. The indexes for the chimera formation ability include the strength of the chimera formation ability or the balance in chimera formation ability between tissues is mentioned. Based on the indexes, a pluripotent cell having a desired chimera formation ability may be selected.

### Examples

### Example 1: Preparation of Prdm14 gene knockout pluripotent cell

In this Example, a Prdm14 gene knockout (disrupted) pluripotent cell was produced.

For determining a chimera rate, an embryonic stem cell (ES cell) having EGFP expressed therein was used as a pluripotent cell. Male and female pluripotent cells are both prepared. More specifically, male and female embryos were obtained by crossing a male of an EGFP transgenic mouse and a C57BL6/N female (all purchased from Japan SLC). Then a male ES cell strain (SGE2) and a female ES cell strain (SGE-F13) were established from the male and female embryos, respectively. In this Example, these ES cell strains will be referred to as wild-type ES cells.

Subsequently, a Prdm14 gene knockout (disrupted) ES cell was prepared from a wild-type ES cell. More specifically, a male Prdm14 gene knockout (disrupted) ES cell (Prdm14-/-ESC-M) was prepared by transfecting an SGE2 cell with gRNA cloning vector (Addgene, Plasmid #41824) having Prdm14 gRNA incorporated therein, together with a Cas9 expression vector (Addgene, Plasmid #41815). A female Prdm14 gene knockout (disrupted) ES cell (Prdm14-/-ESC-F) was prepared in the same manner as in the case of Prdm14-/-ESC-M except that SGE-F13 cell was used in place of the SGE2 cell.

References are given below to illustrate some technical principles which are considered as useful for understanding the invention.

### Reference 2: Preparation of chimeric mouse

A chimeric mouse was prepared by using the Prdm14 gene knockout pluripotent cell prepared in Example 1 and a wild-type pluripotent cell.

ICR mice (purchased from Japan SLC) were crossed to obtain an embryo (morula) on Day 2.5. A male or female-derived Prdm14 gene knockout pluripotent cell or a wild-type pluripotent cell was injected into the cavity of the embryo at a rate of 10 cells per embryo, by use of a micromanipulator under a microscope. Thereafter, the embryo was cultured in KSOM-AA (Millipore) for a day and the resultant fertilized egg in the stage of a blastocyst was transplanted into the uterus of a false pregnancy ICR lineage mouse of Day 2.5 after crossing. On Day 12 after transplantation (on Day 14.5 as fetal age), a non-human fetus was taken out by caesarean section. The non-human fetus obtained was observed by a fluorescence microscope and formation of a chimera was determined based on the fluorescence of EGFP as an index.

In non-human individuals (n = 4) from which fluorescence emission was observed, the chimera rate of each organ was determined. More specifically, from each of E14.5 chimeric mice from which fluorescence emission was observed, the epidermis (MEF), liver, brain and genital ridge were collected. The MEF, genital ridge and brain were cut into pieces by scissors and thereafter treated with 0.025% trypsin-EDTA (Invitrogen) for 10 minutes at 37°C to obtain cell suspensions. The liver was disrupted by pipetting to obtain a cell suspension.

To the liver suspension, 0.5 µl of a mouse CD45-APC (eBio) was added. To a genital ridge cell suspension, 0.5 µl of SSEA1-APC (eBio) antibody was added. These were allowed to stand still for 30 minutes on ice in the dark. The liver, MEF, genital ridge and brain were separately washed with phosphate buffered saline (staining medium; SM), and then, resuspended with 1 µg/ml propidium iodide (PI)-containing phosphate buffered saline, and analyzed by FACSAria II (BD Biosciences) and software, Flow-jo. The chimera rate was determined based on the percentage of a GFP positive cell to the whole cells. The results were as shown in Figure 1.

As shown in Figure 1, in a non-human individual obtained from the embryo when a wild-type pluripotent cell was introduced into an embryo, a GFP positive cell was observed in a certain percentage in any one of the brain, fibroblast, blood (liver) and a germ cell. In contrast, in the cases where a male-derived Prdm14 gene knockout pluripotent cell and a female-derived Prdm14 gene knockout pluripotent cell were used, a GFP positive cell was not observed in the germ cell. From this, it was demonstrated that a Prdm14 gene knockout pluripotent cell contributes to neither the male-derived germ cell and female-derived germ cell.

### Reference 3: Formation of chimera using Otx2 gene knockout pluripotent cell

An Otx2 gene knockout pluripotent stem cell was introduced into an embryo.

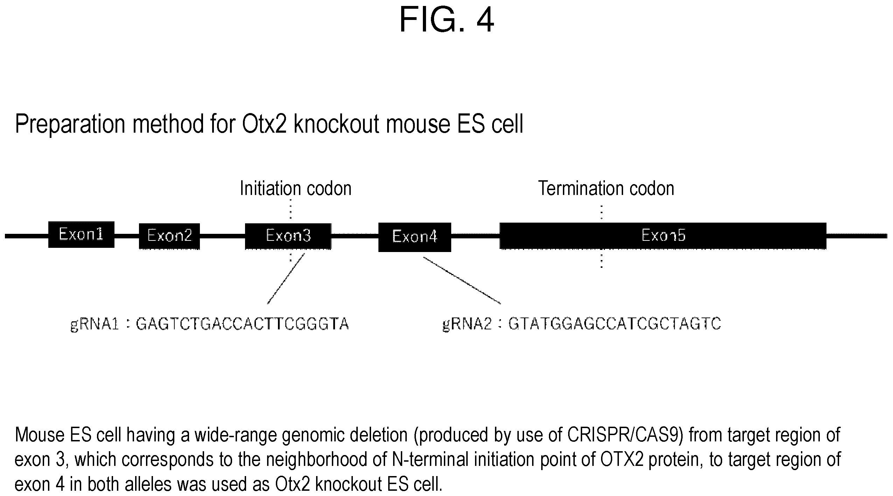

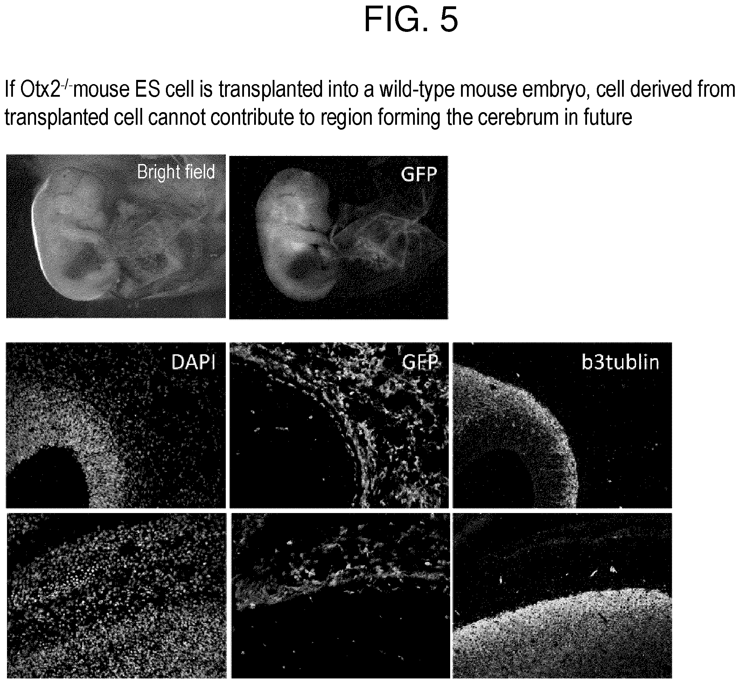

The Otx2 locus of a GFP-expressing mouse ES cell was widely disrupted by use of CRISPR/CAS9. More specifically, a complex of crRNA and tracrRNA, which respectively contain gRNA1 sequence and gRNA2 sequence (shown in Figure 4), and CAS9 protein (all using Alt-R CRISPR-Cas9 system of Integrated DNA Technologies, Inc.) were introduced by electroporation. In the cloned lines, the cell strain where a wide-range sequence deletion occurs from exon 3 to exon 4 in both alleles was determined as Otx2^{-/-}ES cell. Note that, since the ES cell is established from an embryo (B6xBDF1) obtained by crossing B6 lineage mouse and BDF1 lineage mouse, which have black hair color, melanin can be synthesized. The ES cell was introduced into an embryo of a normal mouse (ICR lineage) having white hair. As a result, although transplanted cells were distributed over the whole body based on GFP expression, no accumulation of melanin was observed in the retina (Figure 5, a bright-field image and whole body GFP image). From this, it is considered that Otx2^{-/-}ES cell has a high chimeric contribution ability to the whole body; however, it was demonstrated that Otx2^{-/-}ES cell failed to contribute to the forebrainderived retinal pigment epithelium. Tissue sections of the forebrain region (forming the cerebrum in future) were prepared and immune-stained with an antibody (Abcam, ab18207) against beta-3 tublin, which is a neuroepithelial cell marker, and an anti-GFP antibody (Abcam, Ab13970). As a result, as shown in Figure 5, no contribution of Otx2^{-/-}ES cell-derived cell expressing GFP to the forebrain neuroepithelium (βIII tubulin positive) was observed although the Otx2^{-/-}ES cell-derived cell expressing GFP is present at a high level in other tissues.

As shown in Figure 5, it was found that an Otx2 homo-knockout ES cell loses a differentiation ability into a neuroepithelial cell in the forebrain region including a cell forming the cerebrum in future.

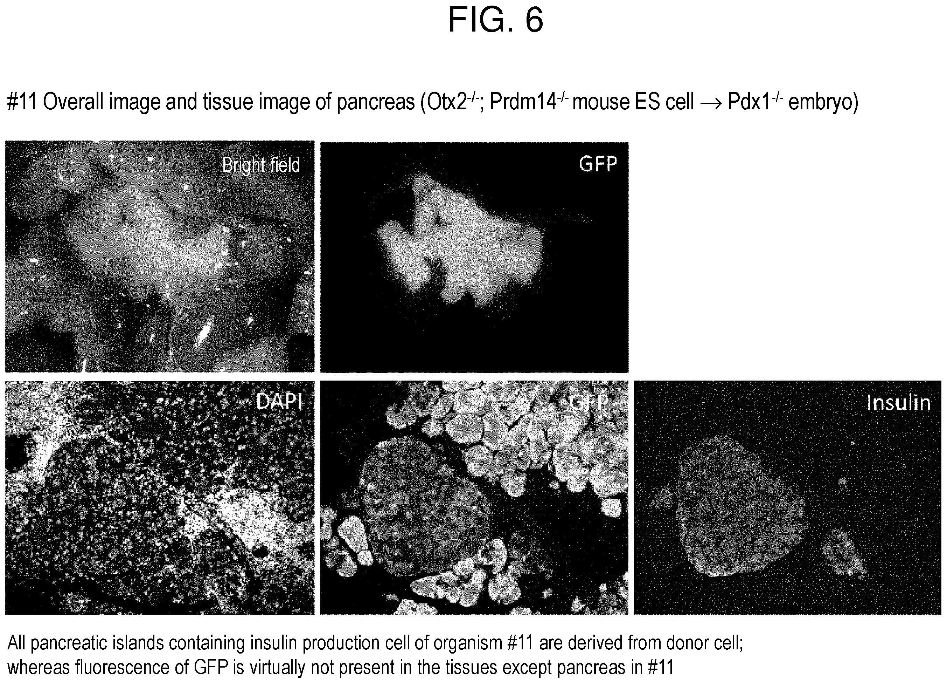

Subsequently, Prdm14 knockout and Otx2 knockout ES cells expressing GFP were introduced into Pdx1-/-knockout embryo, and then, whether the pancreas derived from the ES cells can be formed or not was checked. This is because it is known that a direct target of Otx2 gene contains a Wnt antagonist, Dkk1, which controls Wnt signal (Kimura et al., Developmental Cell 2005), and thus, it is unclear whether the Otx2 knockout cell can normally form an organ or is developed into an organ having a normal function. The results were as shown in Figure 6. As shown in Figure 6, in a chimeric organism (#11) of Pdx1-/-knockout embryo and a Prdm14 knockout and Otx2 knockout ES cell expressing GFP, a pancreas derived from transplanted ES cell (GFP positive) was formed. Tissue sections of the pancreas were prepared and immunostained with an anti-GFP antibody (Abcam, Ab13970) and an antiinsulin antibody (Abcam, Ab7842). As a result, it was confirmed that all pancreatic islets on tissue sections containing an insulin producing cell are derived from a transplanted cell. Since mouse #11 at the time of analysis was 10 weeks after birth, it is supported that the pancreas derived from a transplanted cell normally functions for a long term after birth. Note that, in the tissues except the pancreas tissue in mouse #11, GFP positive cells are virtually not present. The reason is considered that the chimera formation ability of the ES cell introduced in organism #11 was weak.

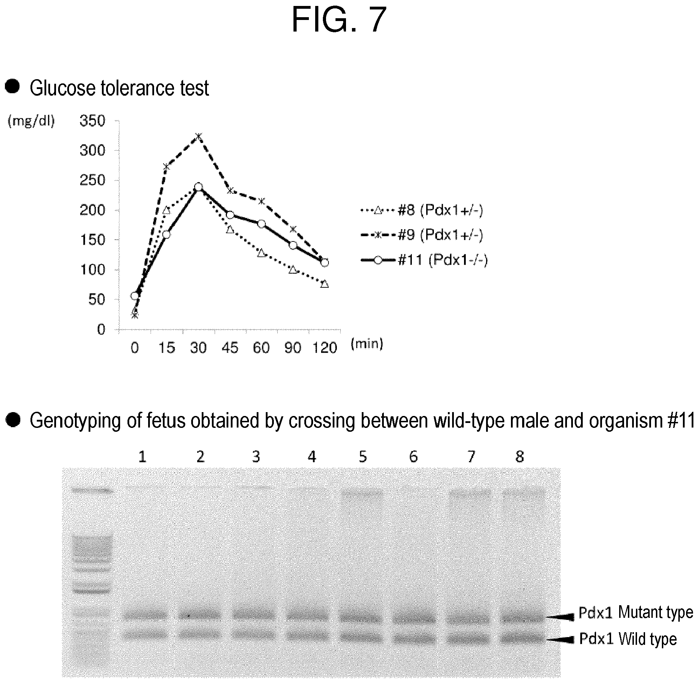

Prior to laparotomy analysis, mouse #11 and littermates (#8, #9) at 8 weeks after birth were subjected to a glucose tolerance test. The results are shown in Figure 7. In the glucose tolerance test, a 150 mg/ml glucose/physiological saline solution was intraperitoneally injected to a mouse at a dose of 10 µL per body weight (1 g) and a change of blood-sugar level with time was measured. As the results of the glucose tolerance test, as shown in Figure 7, it was clear that the pancreas derived from a Prdm14 knockout and Otx2 knockout ES cell formed within a Pdx1-/-knockout embryo has a normal ability to lower blood glucose and is functional.

A mouse #11 (female) was crossed with a wild type mouse to obtain F1 mice. In F1 mice (n = 8), Pdx1 genotype was determined. As a result, as shown in Figure 7, any one of F1 mice has a genotype of Pdx1+/-. This means that all the cells contributing to a germ cell had a genotype of Pdx-/-. In other words, it means that a Prdm14 knockout and Otx2 knockout ES cell (Pdx+/+) does not contribute to a germ cell. Note that, in the Pdx1 locus of the Pdx1 knockout mouse, since e.g., LacZ gene is introduced (Offield et al., Development 1996), a larger gene amplification band than that (405 bp) of the wild type is detected.

As described above, if a Prdm14 gene knockout pluripotent cell was used as the cell to be introduced into a non-human embryo to produce a non-human somatic chimera animal, contribution of the pluripotent stem cell to a germ cell can be reduced or deprived. Also, if an Otx2 gene knockout pluripotent cell was used as the cell to be introduced into a non-human embryo to produce a non-human somatic chimera animal, contribution of the pluripotent stem cell to the cerebral cortex can be reduced or deprived.

### Sequence listing

SEQ ID NO:1: Prdm14 gene targeting sequence of gRNA (GAACCTCGCCACCACCGAGG)
SEQ ID NO:2: Prdm14 gene targeting sequence of gRNA (GTATGGAGCCATCGCTAGTC)
SEQ ID NO:3: A Prdm14 gene sequence knocked out (CTCGCCACCACCG GTCCGGAGCACCCAACCG)
SEQ ID NO:4: A Prdm14 gene sequence knocked out (CTCGCCACCACCG CAGTATTAAAACATGGATGTA)
SEQ ID NO:5: Otx2 gene targeting sequence of gRNA1 (GAGTCTGACCACTTCGGGTA)
SEQ ID NO:6: Otx2 gene targeting sequence of gRNA2 (GTATGGAGCCATCGCTAGTC)
SEQ ID NO:7: Forward primer for Pdx1 amplification (ATTGAGATGAGAACCGGCATG)
SEQ ID NO:8: Reverse primer for wild type Pdx1 amplification (TTCATGCGACGGTTTTGGAAC)
SEQ ID NO:9: Reverse primer for mutant Pdx1 amplification (TGTGAGCGAGTAACAACC)

## Claims

1. A pluripotent cell, wherein the pluripotent cell is genetically engineered not to be differentiated into a predetermined type of cell,
(i) wherein the pluripotent cell has a Prdm14 knockout and an Otx2 knockout; or
(ii) wherein the pluripotent cell has both
a cytotoxic gene, which is operatively connected to an enhancer and/or promoter of a gene exhibiting germ cell specific expression, and
a cytotoxic gene, that is operatively connected to an enhancer and/or promoter of a gene exhibiting forebrain specific expression,
wherein the gene exhibiting germ cell specific expression is Prdm14, and the gene exhibiting forebrain specific expression is Otx1 or Otx2.

2. The pluripotent cell according to claim 1, wherein the pluripotent cell has a Prdm14 knockout and an Otx2 knockout.

3. The pluripotent cell according to claim 1(ii),
wherein the gene exhibiting germ cell specific expression is Prdm14, and the gene exhibiting forebrain specific expression is Otx1 or Otx2.

4. A composition comprising the pluripotent cell according to any one of claims 1 to 3.

## Patentansprüche

1. Pluripotente Zelle, wobei die pluripotente Zelle gentechnisch verändert ist um nicht in einen vorbestimmten Zelltyp differenziert zu werden,
(i) wobei die pluripotente Zelle einen Prdm14-Knockout und einen Otx2-Knockout hat; oder
(ii) wobei die pluripotente Zelle sowohl
ein zytotoxisches Gen hat, das operativ verbunden ist mit einem Enhancer und/oder Promotor eines Gens, das eine Keimzellen-spezifische Expression aufweist, als auch
ein zytotoxisches Gen, das operativ verbunden ist mit einem Enhancer und/oder Promotor eines Gens, das eine Vorderhirn-spezifische Expression aufweist,
wobei das Gen, das eine Keimzellen-spezifische Expression aufweist, Prdml4 ist, und das Gen, das eine Vorderhirn-spezifische Expression aufweist, Otx1 oder Otx2 ist.

2. Die pluripotente Zelle nach Anspruch 1, wobei
die pluripotente Zelle einen Prdm14-Knockout und einen Otx2-Knockout hat.

3. Die pluripotente Zelle nach Anspruch 1(ii),
wobei das Gen, das eine Keimzellen-spezifische Expression aufweist, Prdm14 ist und das Gen, das eine Vorderhirn-spezifische Expression aufweist, Otx1 oder Otx2 ist.

4. Zusammensetzung, die eine pluripotente Zelle nach einem der Ansprüche 1 bis 3 umfasst.

## Revendications

1. Cellule pluripotente, dans laquelle la cellule pluripotente est génétiquement modifiée pour ne pas se différencier en un type prédéterminé de cellule,
(i) dans laquelle la cellule pluripotente présente une inactivation de Prdm14 et une inactivation de Otx2 ; ou
(ii) dans laquelle la cellule pluripotente présente à la fois
un gène cytotoxique, qui est relié de manière fonctionnelle à un amplificateur et/ou à un promoteur d'un gène présentant une expression spécifique de cellules germinales, et
un gène cytotoxique, qui est relié de manière fonctionnelle à un amplificateur et/ou à un promoteur d'un gène présentant une expression spécifique de cerveau antérieur,
dans laquelle le gène présentant une expression spécifique de cellules germinales est Prdm14, et le gène présentant une expression spécifique de cerveau antérieur est Otx1 ou Otx2.

2. Cellule pluripotente selon la revendication 1, dans laquelle la cellule pluripotente présente une inactivation de Prdm14 et une inactivation de Otx2.

3. Cellule pluripotente selon la revendication 1(ii), dans laquelle le gène présentant une expression spécifique de cellules germinales est Prdm14, et le gène présentant une expression spécifique de cerveau antérieur est Otx1 ou Otx2.

4. Composition comprenant la cellule pluripotente selon l'une quelconque des revendications 1 à 3.
